# EUROPEAN PATENT SPECIFICATION

(11) **EP 1 773 288 B1**
(45) Date of publication and mention of the grant of the patent: **24.08.2011**
(21) Application number: 05770629.3
(22) Date of filing: 13.07.2005
(51) Int. Cl.: A61K 8/25, A61K 8/19, A61K 8/29, A61Q 1/12, A61Q 1/02

(54) **COSMETIC POWDER COMPOSITIONS HAVING LARGE PARTICLE SIZE COLOR TRAVEL EFFECT PIGMENTS**
KOSMETIKPUDERZUSAMMENSETZUNGEN MIT FARBFLOP-PIGMENTEN VON GROSSER PARTIKELGRÖSSE
COMPOSITIONS DE POUDRE COSMETIQUE COMPRENANT DES PIGMENTS A GRANDE DIMENSION DES PARTICULES ET COULEUR EVOLUTIVE

(30) Priority: 13.07.2004 US 889003
(43) Date of publication of application: 18.04.2007
(73) Proprietor: Merck Patent GmbH, 64293 Darmstadt (DE)
(72) Inventor: PENG, Qinyun, Yorktown Heights, NY 10598 (US); LINZ, Philip, Croton-Hudson, NY 10520 (US)
(86) International application number: PCT/EP2005/007585
(87) International publication number: WO 2006/005595

(56) References cited:
- EP-A- 1 254 928
- EP-A- 1 281 732
- EP-A- 1 306 412
- EP-A- 1 520 883
- EP-A- 1 555 009
- WO-A-03/006558
- WO-A-2005/094780
- FR-A- 2 845 277
- LINZ P ET AL: "COLOR-TRAVEL COSMETIC PIGMENTS: INTERFERENCE TO THE MAX" COSMETICS & TOILETRIES, WHEATON, IL, US, vol. 118, no. 12, December 2003 (2003-12), pages 63-70, XP008058635 ISSN: 0361-4387

## Description

This invention relates to cosmetic compositions preferably powder compositions having color travel effects.

With the emerging of new pigment technologies, interference pigments with unique, spectacular effects, such as color travel ("color variable"), have been developed. However, the eye-catching color travel pigments of normal particle sizes (1-80 µm) is not readily visible in cosmetic powder products or upon application on the skin when a relatively low concentration of the pigments was used. By low concentration is meant generally about 1-10 percent by weight in the powder composition. If a large quantity of the normal particle size color travel pigments is used, it is likely possible to achieve the color travel effect, but it may not be economically practical with this type of special effect pigments.

An objective of this invention, therefore, is to provide an economical cosmetic formulation having a color travel effect.

Upon further study of the specification, other objects and advantages of the invention will become apparent.

To achieve these objects, there are provided cosmetic compositions preferably in powder form, comprising color travel pigments, wherein said composition contains 0.01-100 % by weight of large particle size color travel pigments to retain the color travel effect in the formulations and upon application to skin.

By using large particle size color travel pigments, it is possible to achieve the color travel effect on the skin using powder applications and the like without using high concentrations of the pigments, unlike their normal particle size counterparts. Here, "large particle size" pigments are defined as having a median particle size (D50) of 40 µm or higher, preferably 60 µm or higher. The normal particle size pigments usually have median particle sizes (D50) less than 40 µm but larger than 5 µm and are disclosed in the prior art, for example in, FR-A-2 845 277, WO 03/006558 A, EP-A-1 254 928, EP-A-1 306 412, EP-A-1 281 732, EP-A-1 520 883 and Linz. P. et al. in Cosmetics & Toiletries, Vol. 118, no. 12, December 2003 (2003-12), pages 63-70: "Color-travel cosmetic pigments: Interference to the max".

The maximum D50 of the large particle size pigments is preferably 150 µm, more preferably about 85 µm with the preferred particle size D50 being 75-95 µm. The particle size of such glitter pigment can reach up to about 500 µm.

One example of the normal particle size pigment is silica based color travel pigments. When 10% of this type of pigment (its particle size range is 10-50 µm, D50 is 16-25 µm) is added to powder formulations, a subtle color travel effect can be seen from the powder cake, but it disappears once the powder is applied onto the skin. On the other hand, if a color travel pigment with large particle size (D50 > 60 µm) is employed, not only the color travel effect can be observed clearly on the powder cake, but it is also retained upon application on the skin. Without being bound by an explanation of the unexpected advantage of using large particle size color travel pigments in powder formulations and their application on skin, at least two factors are believed to be important:

### The presence of light scattering agents:

In a typical powder formulation, talc, kaolin, starch, and magnesium stearate are usually used at high concentration (more than 60% by weight combined, in many cases). Absorption pigments are also commonly used to impart colors (e.g. organic dyes and likes, iron oxides, etc.). The particle sizes of the fillers and absorption pigments are generally less than 25 µm. Their functions are filling, anti-caking or imparting colors. However, they also partially act as light scattering agents due to their high edge to area ratio. In some cases, they may also inevitably decorate the surface of the color travel pigments and consequently reduce the light reflection from the surface of the color travel pigments. The scattering effect (or morphological perturbation) from other ingredients in powder formulations usually reduces or eliminates the color travel effect. However, it has less impact on large particle size pigments and therefore, the luster intensity of large particle size color travel pigment decreases less significantly than that of smaller particle size color travel pigment. The incorporation of at about

0.01-35 %, more preferably about 1-20% by weight of the large color travel pigment particles is generally sufficient to obtain the desired color travel effect, but the specific value may vary dependent on the ingredients of the color travel effect, but the specific value may vary dependent on the ingredients of the powder composition. The upper limit of the concentration of the large particles will be dictated by aesthetic and cost factors.

### Skin property and pigment orientation:

Many skin topology studies have shown that human skin is not completely smooth. It has invisible peaks and valleys, i.e. skin roughness. Some skin roughness data were reported according to different testing methods. They range from a few micrometers to tens of micrometers depending on the testing methods, skin conditions and many other factors. Nonetheless, the skin roughness can be significant enough to interfere with the pigment performance on the skin.

When a powder product is applied onto the skin, the reflectivity of color travel pigment can be further reduced by the skin roughness due to the disorientation of pigment particles and light scattering from the skin. As indicated above, the effect of light scattering from the skin on the large particle size pigments is less pronounced than on the small particle pigments. Hence, our eyes perceive a higher brilliance from the large particle size pigments. Furthermore, large particle size color travel pigments can generally align themselves in parallel better than the smaller ones, owing to their flow characteristics or higher aspect ratio. This again renders a less disturbed light reflection and consequently higher luster intensity and more visible color from the large particle size color travel pigments.

### Possible compositions of the color travel pigments:

One type of color travel pigments described in the present invention are substrate-based pearlescent pigments. Suitable base substrates for the inventive pigments according to the invention are flake-form substrates. Preferred substrates are phyllosilicates. Particularly suitable are natural and/or synthetic mica, aluminum oxides, glass flakes, borosilicate flakes, SiO₂ flakes, talc, kaolin, sericite, flake-form iron oxides or TiO₂ flakes, graphite flakes, BiOCl or other comparable materials.

The size of the base substrates is important per se and can be matched to the particular application. In general, the flake-form substrates have a thickness of between 0.01 and 5 µm, in particular between 0.1 and 4.5 µm. The size in the other two directions is usually between 1 and 550 µm, preferably between 2 and 300 µm, and in particular between 10 and 150 µm (at least 75% within range). The aspect ratio (ratio of surface dimension to thickness of an object) is preferably about 1-500, especially 40-350.

Pigments having color travel effects are defined as exhibiting angle-dependent color change between a number of intense interference colors.

The color travel pigments according to the invention have high and/or low refractive-index layer(s) on top of the surface. The high-refractive-index layer(s) have a refractive index of n > 1.8, preferably of n ≥ 2.0. The high refractive-index layers preferably comprise TiO₂, ZrO₂, SnO₂, ZnO, BiOCl, Fe₂O₃, Fe₃O₄, Cr₂O₃, CeO₃, molybdenum oxides, CoO, Co₃O₄, VO₂, V₂O₃, NiO, V₂O₅, CuO, Cu₂O, Ag₂O, CeO₂, MnO₂, Mn₂O₃, Mn₂O₅, titanium oxynitrides, pseudobrookite, ilmenite, as well as titanium nitride, MoS₂, WS₂ or mixtures or combinations thereof. The TiO₂ here can be in the rutile or anatase modification, preferably in the rutile modification.

Suitable low-refractive-index materials (n ≤ 1.8) are preferably metal oxides or the corresponding oxide hydrates, such as, for example, SiO₂, Al₂O₃, AlO(OH), B₂O₃, MgF₂, MgSiO₃ or a mixture of the said metal oxides.

The layer thickness of the high refractive-index layer is 20 - 500 nm, preferably 50 - 300 nm. The layer thickness of the low refractive-index layer is 20 - 500 nm, preferably 50 - 300 nm. Preferred color travel pigments have three, four or five layers.

Especially preferred color travel pigments show the following layer structure: substrate + high refractive-index layer + low refractive index layer + high refractive index layer.

Particularly interesting color travel pigments have the following layer sequences:

substrate + TiO₂ + SiO₂ + TiO₂

substrate + Fe₂O₃ + SiO₂ + TiO₂

substrate + TiO₂ + SiO₂ + Fe₂O₃

substrate + Fe₂O₃/TiO₂ + SiO₂ + TiO₂

substrate + Fe₂O₃/TiO₂ + SiO₂ + Fe₂O₃/TiO₂

substrate + TiO₂ + SiO₂ + Fe₂O₃/TiO₂

substrate + Fe₃O₄ + SiO₂ + TiO₂

substrate + TiO₂ + SiO₂ + Fe₃O₄

substrate + TiO₂ + Al₂O₃ + TiO₂

substrate + Fe₂O₃ + Al₂O₃ + TiO₂

substrate + TiO₂ + Al₂O₃ + Fe₂O₃

substrate + Fe₂O₃/TiO₂ + Al₂O₃ + TiO₂

substrate + Fe₂O₃/TiO₂ + Al₂O₃ + Fe₂O₃/TiO₂

substrate + TiO₂ + Al₂O₃ + Fe₂O₃/TiO₂

substrate + Fe₃O₄ + Al₂O₃ + TiO₂

substrate + TiO₂ + Al₂O₃ + Fe₃O₄

Additionally on the outer (metal oxide) layer absorption pigments or water insoluble dye(s)/lake(s) (such as ferric ferrocyanide, carmine, organic dyes/lakes such as FD&C dyes/lakes or D&C dyes/lakes, etc.) can be applied.

In the case that the substrate is a glass flake or borosilicate flake the substrate can be coated with a SiO₂ layer first and in the second step with the layer sequence: high refractive layer + low refractive layer + high refractive layer. Preferred pigments based on glass flakes or borosilicate flakes have the following layer structure:

glass flakes + SiO₂ + TiO₂ + SiO₂ + TiO₂

borosilicate flakes + SiO₂ + TiO₂ + SiO₂ + TiO₂

The pigments according to the invention can be prepared relatively easily by the precipitation of high- and low-refractive-index metal oxide layers having precisely defined thickness and a smooth surface on the finely divided, flake-form substrates.

The metal-oxide layers are preferably applied by wet-chemical methods. Methods of this type are described, for example, in DE 14 67 468, DE 19 59 988, DE 20 09 566, DE 22 14 545, DE 22 15 191, DE 22 44 298, DE 23 13 331, DE 25 22 572, DE 31 37 808, DE 31 37 809, DE 31 51 343, DE 31 51 354, DE 31 51 355, DE 32 11 602, DE 32 35 017, EP 1375601 A1 or in other patent documents and other publications known to the person skilled in the art.

In the wet coating method, the substrate particles are suspended in water, and one or more hydrolyzable metal salts are added at a pH which is suitable for hydrolysis and which is selected so that the metal oxides or metal oxide hydrates are precipitated directly onto the flakes without secondary precipitations occurring. The pH is usually kept constant by simultaneous metered addition of a base or acid. The pigments are subsequently separated off, washed and dried and, if desired, calcined, where the calcination temperature can be optimized with respect to the coating present in each case. In general, the calcination temperatures are between 250 and 1000°C, preferably between 350 and 900°C. If desired, the pigments can be separated off after application of individual coatings, dried and, if desired, calcined and then resuspended for the deposition of the further layers.

The coating may furthermore also take place in a fluidized-bed reactor by gasphase coating, it being possible, for example, to use correspondingly the methods proposed in EP 0 045 851 and EP 0 0106 235 for the preparation of color travel pigments.

The production of Ti suboxide or Fe₃O₄ layers can be carried out, for example, by reduction of the TiO₂ layer using ammonia, hydrogen and also hydrocarbons and hydrocarbon/ammonia mixtures, as described, for example, in EP-A-0 332 071, DE 199 51 696 A1 and DE 199 51 697 A1. The reduction is preferably carried out in a forming-gas atmosphere (92% of N₂/8% of H₂ or 96% of N₂/4% of H₂). The reduction is generally carried out at temperatures of 250-1000°C, preferably 350-900°C and in particular 500-850°C.

The hue of the pigments can be varied within broad limits through a different choice of the coating amounts or the layers resulting therefrom. Fine tuning for a certain hue can be achieved beyond the pure choice of amount by approaching the desired color under visual or measurement technology control.

Furthermore, organic or combined organic/inorganic post-coatings are possible, for example with silanes, as described, for example, in EP 0090259, EP 0 634 459, WO 99/57204, WO 96/32446, WO 99/57204, U.S. 5,759,255, U.S. 5,571,851, WO 01/92425 or in J.J. Ponjeé, Philips Technical Review, Vol. 44, No. 3, 81 ff. and P.H. Harding J.C. Berg, J. Adhesion Sci. Technol. Vol. 11 No. 4, pp. 471-493.

The pigments of the present invention can also advantageously be used in blends with organic dyes, organic pigments or other pigments, such as, for example, transparent and opaque white, colored and black pigments, and with flake-form iron oxides, organic pigments, holographic pigments, LCPs (liquid crystal polymers) and conventional transparent, colored and black luster pigments based on metal oxide-coated mica and SiO₂ flakes, etc. The color travel pigments can be mixed in any ratio with commercially available pigments and fillers.

As for the nature of the color travel pigments, all types which exhibit a color travel effect can be used in the present invention. More examples of such pigments include but are not limited to those described in EP 1375601 A1, as well as to those described in the patents and literature cited therein, e.g. U.S. 4,434,010, JP H7-759, U.S. 3,438,796, U.S. 5,135,812, DE 44 05 492, DE 44 37 753, DE 195 16 181 and DE 195 15 988, DE 196 18 569, DE 197 46 067 and in the literature, for example in EURO COSMETICS, 1999, No. 8, p. 284.

The compositions of this invention are preferably in the form of a cosmetic powder for application to skin. Examples of such cosmetic powders include but are not limited to: eye shadow, blusher, powder makeup, lip powder, face powder, body powder, bronzing powder.

Aside from powders, it is contemplated that the powders can be incorporated in various systems so as to form formulations such as for example foundation (liquid and stick), face makeup such as cream-to-powder, eye highlighter, eye pencil, bronzing stick, etc.

Also, the large particle size color travel pigments of this invention will exhibit a sparkling effect and appear to be more lustrous than the normal particle size color travel pigments when dispersed in a wax base or fluid system or the like, such as, for example, in lip gloss, lipstick, nail polish, eyeliner, mascara, hair gel, shower gel, body lotion, skin cream, shampoo, etc.

To reiterate, important aspects of this invention, include but are not limited to color travel pigments having a large particle size (D50 > 60 µm), and coated with two layers or more, especially with alternating layers of high and low refractive index, for example, with TiO₂-SiO₂-TiO₂. The preferred coatings are with metal oxides, preferably TiO₂ and SiO₂ and/or Fe₂O₃, with the TiO₂ being rutile or anatase, preferably rutile. The large particle size color travel pigments of the invention can be modified with a coating of absorption pigments or water insoluble organic dye/lakes on top, for example without exclusion ferric ferrocyanide, Prussian Blue, Indigo, Carmine, FD&C dyes and lakes, and D&C dyes and lakes. A protective layer can also be provided on the modified or unmodified color travel pigment. The substrate of the color travel pigments include, but are not limited to: mica, SiO₂ flakes, Al₂O₃ flakes, glass flakes, borosilicate flakes, graphite flakes, and BiOCl.

The desired particle sizes of the pigments are obtained by conventional methods, e.g. sieving or sedimentation.

It goes without saying that, for the various applications, the color travel pigments can also advantageously be used in the form of a mixture with organic dyes, organic pigments or other pigments, such as, for example, transparent and opaque white, coloured and black pigments, and with flake-form iron oxides, organic pigments, holographic pigments, LCPs (liquid crystal polymers) and conventional transparent, coloured and black lustre pigments based on metal oxide-coated mica and SiO₂ flakes, etc. The color travel pigments can be mixed in any ratio with commercially available pigments and fillers.

In the various applications, the pigment according to the invention can also be combined with further colorants of any type, for example organic and/or inorganic absorption pigments and dyes, multilayered interference pigments, such as, for example, Timiron^{®} (Merck KGaA, Rona), Sicopearl^{®} (BASF AG), ChromaFlair^{®} (Flex Products Inc.), BiOCl pigments, pearl essence or coated or uncoated metal pigments, for example from Eckart. There are no limits to the mixing ratios and concentration.

The pigments according to the invention are compatible with a multiplicity of colour systems, preferably from the areas of plastics, paints, powder coatings, coatings and printing inks and especially preferred in cosmetic formulations.

The color travel pigments and pigment mixtures have particular applications in decorative and personal care cosmetic preparations, especially in powder form. The use concentration ranges from 0.01 % by weight in shampoo to 100% by weight in the case of loose powders. In the case of a mixture of the color travel pigments with spherical fillers, for example SiO₂, the concentration in the formulation can be 0.01-70% by weight. The cosmetic products, such as, for example, nail varnishes, compact powders, shampoos, loose powders and gels, are distinguished by particularly interesting color effects and an intense color shift. The color flop effect in nail varnish can be significantly increased compared with conventional nail varnishes with the aid of the pigments according to the invention.

The formulations containing the color travel pigments according to the invention can belong to the lipophilic, hydrophilic or hydrophobic type. In the case of heterogeneous formulations having discrete aqueous and non-aqueous phases, the color travel pigments according to the invention may in each case be present in only one of the two phases or alternatively distributed over both phases.

Without further elaboration, it is believed that one skilled in the art can, using the preceding description, utilize the present invention to its fullest extent. The following preferred specific embodiments are, therefore, to be construed as merely illustrative, and not limitative of the remainder of the disclosure in any way whatsoever.

In the foregoing and in the following examples, all temperatures are set forth uncorrected in degrees Celsius and, all parts and percentages are by weight, unless otherwise indicated.

As a disclaimer, it is to be noted that one or more examples may have not been actually conducted.

### Example 1: Color travel pigment

100 g of mica (particle size 10-150 µm, D50 = 75-95 µm) is suspended in deionized water and the suspension is heated to 75°C with stirring. When this temperature has been reached, a solution consisting of 3 g of SnCl₄ x 5 H₂O and 10 ml of hydrochloric acid (37%) in 90 g of water is slowly metered into the mica suspension with vigorous stirring. 220 ml of TiCl₄ solution (400 g of TiCl₄/l) are metered in at a pH of 2.0. The pH is subsequently raised to 7.0, and 830 ml of sodium water-glass solution (13% by weight of SiO₂) is slowly metered in at this pH, during which the pH is kept constant at 7.5 using 10% HCl. A solution of 11.5 g of SnCl₄ x 5 H₂O and 10 ml of hydrochloric acid (32%) in 350 ml of deionised water is subsequently metered in at pH 1.8. Finally, 220 ml of TiCl₄ solution (400 g of TiCl₄/l) are metered in at pH 2.0. During the addition of the SnCl₄ x 5 H₂O solutions and TiCl₄ solutions, the pH is in each case kept constant using NaOH solution (32%).

For work-up, the coated mica pigment is filtered off, washed and dried at 110°C for 16 hours. Finally, the pigment is calcined at 800°C for 0.5 hour.

The pigment thus obtained when viewed straight on exhibits an intense blue-green interference color which changes through violet into red when the pigment is tilted.

### Use Examples 1-4

| **LOOSE EYE SHADOW POWDER WITH COLOR TRAVEL PIGMENTS** | | |
|---|---|---|
| **INCI NAME** | **TRADE NAME/MANUFACTURER** | **% by weight** |
| **Phase A** | | |
| Talc | Supra H/Luzenac | q.s. to 100.00 |
| Kaolin | Kaolin, Colloidal, USP/Whittaker, Clark & Daniels | 4.0 |
| Magnesium Stearate | Magnesium Stearate Vegetable F.G./Whittaker, Clark & Daniels | 2.00 |
| Bismuth Oxychloride | Biron^{®} LF-2000/Rona | 5.00 |

| **Phase B** | | |
|---|---|---|
| Iron Oxides, Mica | Colorona^{®} Aborigine Amber/Rona | 7.00 |
| Silica, CI77891 (Titanium dioxide),Mica, Tin oxide | Color Travel Pigment according to Example 1 | |

| **Phase C** | | |
|---|---|---|
| Octyldodecanol | Eutanol G/Cognis | 5.00 |
| **Total** | | **100.00** |

**Procedure**: Combine Phase A. Add Phase B with gentle agitation. Spray Phase C onto batch while agitating bulk. Pass entire batch through a jump gap.

Several examples are prepared, substituting the specific color travel pigment into the above formula:

| Example | Wt% | *Description | D50 |
|---|---|---|---|
| 1 | 25% | Color Travel Pigment | ~ 86 INVENTION |
| 2 | 25% | Xirona^{®} Magic Mauve | 16-22 |
| 3 | 10% | Color Travel Pigment | ~ 86 INVENTION |
| 4 | 10% | Xirona^{®} Magic Mauve | 16-22 |

### INCI Name:

### Xirona^{®} Magic Mauve: silica, titanium dioxide, tin oxide

### Examples 5-8

| **EYE SHADOW WITH COLOR TRAVEL PIGMENTS** | | |
|---|---|---|
| **INCI NAME** | **TRADE NAME/MANUFACTURER** | **%** |
| **Phase A** | | |
| Talc | Supra H/Luzenac | q.s. to 100.00 |
| Aluminium Starch Octenyl | Dry Flo PC/National Starch & | 7.30 |
| Succinate | Chemical | |
| Magnesium Stearate | Magnesium Stearate Vegetable F.G./Whittaker, Clark & Daniels | 2.45 |
| Bismuth Oxychloride | Biron^{®} LF-2000/Rona | 7.00 |

| **Phase B** | | |
|---|---|---|
| Iron Oxides, Mica | Colorona^{®} Aborigine Amber/Rona | 7.00 |
| | Color Travel Pigment according to Example 1 | |

| **Phase C** | | |
|---|---|---|
| Isopropyl Palmitate, | Lexol 3975/Inolex | 10.50 |
| Isopriopyl Myristate, | | |
| Isopropyl Sterate | | |
| Cetyl Palmitate | Cutina CP/Henkel | 0.70 |
| Petrolatum | Snow White Petrolatum/Penreco | 0.70 |
| Propylparaben | Propylparaben/Spectrum Chemical | 0.10 |
| **Total** | | **100.00** |

### Procedure:

Combine ingredients in Phase A. Pulverize with a hammer mill, passing twice through a 0.27" herring bone screen. Add Phase B with gentle agitation. Combine Phase C; heat to 70°C. Solution should be clear and uniform. Spray Phase C onto batch while agitating bulk. Pass entire batch through a jump gap.

Several examples are prepared, substituting the specific color travel pigment into the above formula:

| Example | Wt% | *Description | D50 |
|---|---|---|---|
| 5 | 25% | Color Travel Pigment | ∼86 INVENTION |
| 6 | 25% | Xirona^{®} Magic Mauve | 16-22 |
| 7 | 10% | Large particle size color travel (Red/Gold) | ∼86 INVENTION |
| 8 | 10% | Color Travel Pigment | 16-22 |

### INCI Name:

### Xirona^{®} Magic Mauve: silica, titanium dioxide, tin oxide

The above examples showed that when 10% of normal particle size color travel pigment (Xirona^{®} Magic Mauve: D50 = 16-22) is used, the color travel effect is merely visible in the powder samples and not visible upon application onto skin. However, the color travel effect can be seen clearly in the powder samples containing the large particle size color travel pigment according to Example 1 and when the powders are applied onto skin.

### Example 9 (INVENTION)

| **4-IN-1 POWDER MAKEUP (FOR EYES, CHEEKS, LIPS AND NAILS)** | | |
|---|---|---|
| **INCI NAME** | **TRADE NAME/MANUFACTURER** | **%** |
| **Phase A** | | |
| Mica, Titanium Dioxide; | Color Travel Pigment according to | 35.00 |
| Iron Oxides, Silica | Example 1 | |
| Bismuth Oxychloride | Biron^{®} LF-2000/Rona | 5.00 |
| Talc | Supra H/Luzenac | 38.00 |
| Kaolin | Kaolin, Colloidal, USP/Whittaker, Clark & Daniels | 5.00 |
| Magnesium Stearate | Magnesium Stearate Vegetable F.G./ Whittaker, Clark & Daniels | 2.50 |
| Calcium Silicate | Hubersorb 600/Huber | 0.50 |

| **Phase B** | | |
|---|---|---|
| Mineral Oil (and) Lanolin | Amerchol L-101/Amerchol | 11.90 |
| Alcohol | | |
| Lanolin Alcohol | Super Hartolan/Croda | 1.00 |
| Petrolatum | Snow White Petrolatum/Penreco | 1.00 |
| Propylparaben | Propylparaben/Spectrum Chemical | 0.10 |
| **Total** | | **100.00** |

**Procedure:** Mix the ingredients of Phase A homogeneously. Combine ingredients in Phase B and heat to 80° C with mixing. Add the melted Phase B to Phase A with stirring. The powder is pressed at 40-50 bar (-600 - 700 lb./sq. in.).
**Application**: Apply to eyelids and cheeks and blend gently with fingertips. Outline lips with contour pencil and fill in with color. For nails, apply powder to nails with fingertips and fix with clear nail lacquer. Excess powder on cuticles can easily be washed off.

In the following non-limiting examples 10-18, the invention can be realized by adding or substituting a sufficient amount of any of the exemplified large particle size color travel pigments having a D50 particle size of at least 40 µm, preferably at least 60 µm, to each product. For example, the eye highlighter of example 14 can be made by adding 25% by weight of large particle size color travel (Red/Gold) having a D50 of about 86. The remaining components of example 14 will then amount to 75% by weight of the Eye Highlighter.

### Example 10

| **FOUNDATION** | | |
|---|---|---|
| **INCI NAME** | **TRADE NAME/MANUFACTURER** | **%** |
| **Phase A** | | |
| Water | Water, Demineralized | 50.32 |
| Hydroxylated Lecithin | Alcolec Z-3/American Lecithin Co. | 0.10 |

| **Phase B** | | |
|---|---|---|
| Mica | Mica M/Rona | 5.00 |
| Titanium Dioxide, Mica, Zinc | Microna^{□} Matte White/Rona | 4.00 |
| Oxide | | |
| Mica, Iron Oxides | Microna^{®} Matte Yellow/Rona | 2.00 |
| Mica, Iron Oxides | Microna^{®} Matte Orange/Rona | 0.20 |
| Iron Oxides, Mica | Microna^{®} Matte Red/Rona | 0.20 |
| Iron Oxides, Mica | Microna^{®} Matte Black/Rona | 0.20 |

| **Phase C** | | |
|---|---|---|
| Propylene Glycol | Propylene Glycol/Lyondell | 4.00 |
| Magnesium Aluminum Silicate | Veegum/RT Vanderbilt | 1.00 |

| **Phase D** | | |
|---|---|---|
| Propylene Glycol | Propylene Glycol/Lyondell | 4.00 |
| Cellulose Gum | CMC 7H3SF/Aqualon | 0.15 |

| **Phase E** | | |
|---|---|---|
| Deionized Water | Deionized Water | 5.00 |
| Triethanolamine 99% | Triethanolamine 99%/Dow | 1.00 |

| **Phase F** | | |
|---|---|---|
| Sucrose Cocoate | Crodesta SL40/Croda | 1.50 |
| Methyl Paraben | Methylparaben/Spectrum Chemical | 0.20 |
| Disodium EDTA | Hamp-ene Na2/Hampshire | 0.05 |

| **Phase G** | | |
|---|---|---|
| Propylene Glycol Dicapiylate | Myritol PC/Cognis | 11.00 |
| Dicaprate | | |
| Isostearyl Stearoyl Stearate | Hetester ISS/Heterene | 2.00 |
| Sorbitan Monolaurate | Arlacel 20/Uniquema | 2.50 |
| Cetyl Alcohol | Crodacol C-70/Croda | 1.25 |
| Stearic Acid | Emersol 120/Cognis | 1.50 |
| Oleic Acid | Emersol 210/Cognis | 0.50 |
| BHA | Tenox BHA/Eastman | 0.05 |
| Propyl Paraben | Propylparaben/Spectrum Chemical | 0.10 |

| **Phase H** | | |
|---|---|---|
| DMDM Hydantoin 55% | Glydant/Lonza | 0.18 |
| Water | Water, Demineralized | 2.00 |
| **Total** | | **100.00** |

### Procedure:

**Water Phase**: Combine the ingredients of Phase A. Mix until homogenous. Add Phase B with stirring, mixing until no undispersed pigment remains. Combine and add Phase C. Continue mixing and heat to 90°C for 15 minutes. Cool to 75°C. Combine and add Phase D and Phase E, maintaining agitation until no undispersed particles remain. Add Phase F.
**Oil Phase**: Combine the ingredients of phase G separately from the water phase. Heat to 75-80°C with propeller agitation until homogenous.
**Emulsification**: Add the oil phase to the water phase at 75-80°C, with propeller agitation. Maintain temperature and homogenize for 15 minutes. Cool to 45°C with moderate agitation. Combine and add Phase H. Cool to 30°C.

### Example 11

| **SPF 4.5 LOOSE BODY POWDER** | | |
|---|---|---|
| **INCI Name** | **Trade Name (Supplier)** | **% w/w** |
| **Phase A** | | |
| Talc | Supra H/Luzenac America | 40.50 |
| Mica | Mica M/Rona | 27.50 |
| Mica, Silica | Micronasphere^{®} M/Rona | 10.00 |
| Kaolin | Kaolin, Colloidal, USP/ Whittaker, Clark & Daniels | 4.00 |
| Magnesium Stearate | Magnesium Stearate Vegetable F. G./ Whittaker, Clark & Daniels | 5.00 |
| Titanium Dioxide, Alumina, | Eusolex^{®} T-2000/Rona | 5.00 |
| Simethicone | | |
| Mica, Iron Oxides | Microna^{®} Matte Orange/Rona | 4.34 |
| Iron Oxides, Mica | Microna^{®} Matte Red/Rona | 0.84 |
| Mica, Iron Oxides | Microna^{®} Matte Yellow/Rona | 0.84 |
| Mica, Iron Oxides | Microna^{®} Matte Black/Rona | 0.98 |
| Titanium Dioxide, Mica, Zinc Oxide | Microna^{®} Matte White/Rona | 1.00 |
| **Total** | | **100.00** |

### Procedure:

The powder ingredients are mixed homogeneously and then sieved through a 63 µm screen.

**Note:**
In vivo SPF 4.5 (5 subjects), Colipa Task Force Method

### Example 12

| **LIP POWDER** | | |
|---|---|---|
| **INCI Name** | **Trade Name /Manufacturer** | **%** |
| **Phase A** | | |
| Mica, Iron Oxides | Colorona^{®} Bordeaux/Rona | 30.00 |
| Bismuth Oxychloride | Biron^{®} LF-2000 or Biron^{®} MTU/Rona | 10.00 |
| Talc | Supra Talc/Luzenac | 40.00 |
| Magnesium Stearate | Magnesium Stearate Vegetable Food Grade/ Whittaker, Clark & Daniels | 5.00 |

| **Phase B** | | 5.00 |
|---|---|---|
| Isopropyl Stearate | Tegosoft S/Goldschmidt | 11.20 |
| Dimethicone, Dimethiconol | 1403 Fluid/Dow Corning | 3.75 |
| Fragrance | Fragrance | q.s. |
| Propylparaben | Propylparaben/Sutton | 0.05 |
| **Total** | | **100.00** |

**Procedure**: Mix the ingredients of Phase A homogeneously. Heat and mix the ingredients in Phase B. Add Phase B to Phase A with mixing. The powder is pressed between 40 - 50 bar.

### Example 13

| **EYE PENCIL** | | |
|---|---|---|
| **INCI Name** | **Trade Name /Manufacturer** | **%** |
| **Phase A** | | |
| Italian Talc | Supra H/Luzenac | 11.30 |
| C18-36 Ethylene Glycol Ester | Syncrowax ERL-C/Croda | 8.10 |
| Glyceryl Tribehenate | Syncrowax HR-C/Croda | 1.90 |
| Ethyl Hexyl Palmitate | Pelemol OP/Phoenix Chemical | 38.40 |
| Caprylic/Capric Triglyceride | Myritol 318/Cognis | 4.00 |
| Stearic Acid | Emersol 120/Cognis | 3.00 |
| Polyglyceryl 3-Diisostearate | Emerest 2452/Cognis | 0.50 |
| Methylparaben | Methylpapraben/Spectrum Chemical | 0.20 |
| Propylparaben | Propylparaben/Spectrum Chemical | 0.10 |

| **Phase B** | | |
|---|---|---|
| Bismuth Oxychloride | Biron^{®} MTU/Rona | 15.00 |
| Iron Oxides, Mica | Microna^{®} Matte Red/Rona | 16.00 |
| Iron Oxides, Mica | Microna^{®} Matte Black/Rona | 1.50 |
| **Total** | | **100.00** |

**Procedure**: Combine all ingredients in Phase A and heat to 80 - 85°C with stirring until homogenous. Add Phase B. Agitate with a high-speed mixer until no agglomerates remain. Pour at 70°C.

### Example 14

| **EYE HIGHLITGHTER** | | |
|---|---|---|
| **INCI Name** | **Trade Name /Manufacturer** | **%** |
| **Phase A** | | |
| Talc | Supra H/Luzenac | 48.25 |
| Starch | Novation 1600/National Starch & Chemical | 7.30 |
| Magnesium Stearate | Magnesium Stearate Vegetable F.G./WC&D | 2.45 |
| Bismuth Oxychloride | Biron^{®} LF-2000/Rona | 5.70 |

| **Phase B** | | |
|---|---|---|
| Mica, Titanium Dioxide | Timiron^{®} MP-99/Rona | 17.00 |
| Mica, Titanium Dioxide, Carmine | Colorona^{®} Magenta/Rona | 4.90 |
| Mica, Titanium Dioxide, Iron Oxides | Colorona^{®} Red Gold/Rona | 2.40 |

| **Phase C** | | |
|---|---|---|
| Isopropyl Stearate | Tegosoft S/Goldschmidt | 10.50 |
| Cetyl Palmitate | Cutina CP/Cosgnis | 0.70 |
| Petrolatum | Snow White Petrolatum/Penreco | 0.70 |
| Propylparaben | Propylparaben/Spectrum Chemical | 0.10 |
| **Total** | | **100.00** |

**Procedure**: Combine Phase A. Pulverize with a hammer mill, passing twice through a 0.027" herring bone screen. Add Phase B with gentle agitation. Combine Phase C. Heat to 70°C. Spray onto batch while agitating bulk. Pass entire batch through a jump gap.

### Example 15

| **BRONZING POWDER** | | |
|---|---|---|
| **INCI NAME** | **TRADE NAME/MANUFACTURER** | **%** |
| **Phase A** | | |
| Talc | Supra H/Luzenac | q.s. to 100.00 |
| Lithium Stearate | Lithium Stearate #306/Witco | 3.50 |
| Kaolin | Kaolin 2457/ Whittaker, Clark & Daniels | 5.00 |
| Calcium Silicate | Microcel E/Celite | 0.30 |
| Methylparaben | Methylparaben/Spectrum Chemical | 0.20 |
| Propylparaben | Propylparaben/Spectrum Chemical | 0.10 |
| Bismuth Oxychloride | Biron^{®} MTU/Rona | 5.00 |

| **Phase B** | | |
|---|---|---|
| Mica (and) Iron Oxides | Colorona^{®} Bronze/Rona | 50.00 |

| **Phase C** | | |
|---|---|---|
| Isopropyl Palmitate | Lexol IPP/Inolex | 7.30 |
| Cetyl Palmitate | Cutina CP/Cognis | 2.00 |
| Petrolatum | White Petrolatum/Witco | 2.00 |
| Polyglyceryl-3 | Emerest 2452/Cognis | 0.50 |
| Diisostearate | | |

| **Phase D** | | |
|---|---|---|
| Fragrance | Grapefruit Fragrance 26125V/Shaw Mudge | 0.10 |
| **Total** | | **100.00** |

**Procedure:** Combine Phase A. Pulverize with a hammer mill, passing twice through a 0.027" herring bone screen. Add Phase B with gentle agitation. Combine Phase C. Heat to 70°C, stirring until clear. Reduce temperature to 55-60°C. Add Phase D (if desired). Spray onto batch while agitating bulk. Pass entire batch through a jump gap.

### Example 16

| **FACE POWDER** | | |
|---|---|---|
| **INCI NAME** | **TRADE NAME/MANUFACTURER** | **%** |
| Talc | Supra H/Luzenac America | 87.10 |
| Aluminum Starch | Dry Flo Pure/National Starch | 11.00 |
| Octenylsuccinate | | |
| Mica (and) Iron Oxides | Microna^{®} Matte Yellow/Rona | 1.00 |
| Mica (and) Iron Oxides | Microna^{®} Matte Orange/Rona | 0.20 |
| Iron Oxides (and) Mica | Microna^{®} Matte Red/Rona | 0.20 |
| Iron Oxides (and) Mica | Microna^{®} Matte Black/Rona | 0.20 |
| Methylparaben | Methylparaben/Spectrum Chemical | 0.10 |
| Propylparaben | Propylparaben/Spectrum Chemical | 0.10 |
| Imidazolidinyl Urea | Germall 115/Sutton | 0.10 |
| **Total** | | **100.00** |

**Procedure**: Add ingredients to powder blender. Mix until uniform. Micropulverize. Package.

### Example 17

| **CREAM TO POWDER MAKEUP** | | |
|---|---|---|
| **INCI NAME** | **TRADE NAME/MANUFACTURER** | **%** |
| **Phase A** | | |
| Silica (and) Titanium | Ronasphere^{®} LDP/Rona | 10.00 |
| Dioxide (and) Iron | | |
| Oxides | | |
| Mica (and) Titanium | Extender W/Rona | 12.00 |
| Dioxide | | |
| Mica (and) Iron Oxides | Microna^{®} Matte Yellow/Rona | 4.40 |
| Iron Oxides (and) Mica | Microna^{®} Matte Red/Rona | 0.80 |
| Iron Oxides (and) Mica | Microna^{®} Matte Black/Rona | 0.40 |
| Mica (and) Iron Oxides | Microna^{®} Matte Orange/Rona | 0.40 |
| Talc | Supra H/Luzenac America | 6.40 |
| Silica | Ronasphere^{®} /Rona | 2.10 |
| Aluminum Starch | Dry Flo PC/National Starch | 14.40 |
| Octenyl Succinate | &Chemical | |

| **Phase B** | | |
|---|---|---|
| Octyl Palmitate | Pelemol OP/Phoenix | 29.50 |
| Mineral Oil (and) | Amerchol L-101/Amerchol | 2.50 |
| Lanolin Alcohol | | |
| Copemica Cerifa | Carnauba Wax/Ross | 1.30 |
| (Camauba) Wax | | |
| Ceresin | Ceresin Wax #375/Ross | 2.00 |
| Hydrogenared Castor Oil | Cutina HR/Henkel | 3.50 |
| Phenyl Trimethicone | Dow Coming 556/Dow Coming | 9.50 |
| Sorbitan Sesquioleate | Arlacel 83/Uniquema | 0.50 |
| Propylparaben | Propylparaben/Spectrum Chemical | 0.20 |
| PEG-8 (and) Tocopherol | Oxynex^{®} K/Rona | 0.10 |
| (and) Ascorbyl Palmitate | | |
| (and) Ascorbic Acid | | |
| (and) Citric Acid | | |
| **Total** | | **100.00** |

### Procedure:

Heat the ingredients of Phase B to 85°C with mixing. Blend Phase A and add to Phase B. Continue mixing until the melt is homogeneous. Cool down to 80°C and pour into molds.

### Example 18

| **BLUSHER** | | |
|---|---|---|
| **INCI NAME** | **TRADE NAME/MANUFACTURER** | **%** |
| **Phase A** | | |
| Octyl Palmitate | Pelemol OP/Phoenix Chemical | 29.00 |
| Ozokerite | Ozokerite Wax 77W/Ross | 4.00 |
| Hydrogenated Castor Oil | Cutina HR/Cognis | 4.00 |
| Copernica Cerifa (Camauba) | Carnauba Wax/Ross | 1.00 |
| Wax | | |
| Phenyl Trimethicone | Dow Coming 556 Fluid/Dow Coming | 8.50 |
| Mineral Oil (and) Lanolin | Amerchol L-101/Amerchol | 2.50 |
| Alcohol | | |
| Sorbitan Sesquioleate | Arlacel 83/Uniquema | 0.50 |
| Propylparaben | Propylparaben/Spectrum Chemical | 0.20 |
| Allantoin | Allantoin/Rona | 0.20 |
| PEG-8 (and) Tocopherol (and) | Oxynex^{®} K/Rona | 0.10 |
| Ascorbyl Palmitate (and) | | |
| Ascorbic Acid (and) Citric Acid | | |

| **Phase B** | | |
|---|---|---|
| Talc | Supra H/Luzenac America | 18.60 |
| Aluminum Starch Octenyl | Dry Flo PC/National Starch & | 14.90 |
| Succinate | Chemical | |
| Bismuth Oxychloride | Biron^{®} MTU/Rona | 12.50 |

| **Phase C** | | |
|---|---|---|
| Mica (and) Iron Oxides | Colorona^{®} Bordeaux/Rona | 3.00 |
| Mica (and) Titanium Dioxide | Colorona^{®} Carmine Red/Rona | 1.00 |
| (and) Carmine | | |
| Total | | 100.00 |

**Procedure**: Combine all ingredients in Phase A. Heat to 80-85°C and mix until homogenous. Add Phase B and Phase C with mixing. Pour at 70°C.

These cosmetic compositions can be applied to skin in the conventional manner.

## Claims

1. A cosmetic composition containing pigments having a color travel effect, wherein said composition contains 0.01-35 % by weight, of large particle size color travel pigments having a D50 particle size of at least 60 µm up to 150 µm to retain the color travel effect upon application to skin and wherein the color travel pigments comprise substrates selected from the group consisting of natural and/or synthetic mica, aluminum oxides, glass flakes, SiO₂ flakes, talc, kaolin, sericite, flake-form iron oxides or TiO₂ flakes, graphite flakes, and BiOCl coated with alternating layers of high refractive index coatings having a refractive index of n > 1.8, and low refractive index coatings having a refractive index of n ≤ 1.8.

2. A cosmetic composition according to claim 1, containing 1-20% by weight of the large particle size color travel pigments.

3. A cosmetic coating composition according to any of claims 1 to 2, wherein the alternating layers are metal oxides.

4. A cosmetic composition according to claim 3, wherein the metal oxides are TiO₂ and SiO₂ or Fe₂O₃.

5. A cosmetic composition according to any of claims 1 to 4, wherein the large particle size color travel pigments contain more than 2 layers of metal oxides.

6. A cosmetic composition according to any of claims 3 to 5, wherein said metal oxide layers are TiO₂-SiO₂-TiO₂.

7. A cosmetic composition according to any of claims 4 to 6, wherein the TiO₂ is rutile.

8. A cosmetic composition according to any of claims 1 to 7, wherein the pigments having a color travel effect further comprise as a top layer an absorption pigment or a water insoluble dye(s)/lakes(s).

9. A cosmetic composition according to claim 8, comprising as a top layer a ferric ferrocyanide, Prussian Blue, carmine red, indigo, or carmine, FD&C dyes/lakes, D&C dyes/lakes.

10. A color travel pigment having a D50 particle size of at least 60 µm up to 150 µm and substrates selected from the group consisting of natural and/or synthetic mica, aluminum oxides, glass flakes, SiO₂ flakes, talc, kaolin, sericite, flake-form iron oxides or TiO₂ flakes, graphite flakes, and BiOCl coated with alternating layers of high refractive index coatings having a refractive index of n > 1.8, and low refractive index coatings having a refractive index of n ≤ 1.8.

11. A color travel pigment according to claim 10, having a D50 range of 75-95 µm.

12. A color travel pigment according to any of claims 10 or 11, wherein said pigment comprises as a top layer an absorption pigment, a water insoluble dye(s) or a water insoluble lake(s).

13. A cosmetic composition according to any of claims 1-9, in the form of an eye pencil or eye highlighter, a cream to powder, a liquid or stick foundation or bronzer, lip gloss, lipstick, nail polish, eyeliner, mascara, hair gel, shower gel, body lotion, skin cream or shampoo.

## Patentansprüche

1. Kosmetische Zubereitung, die Pigmente mit Farbwechseleffekt enthält, wobei die Zubereitung 0,01-35 Gew.-% großkörniger Farbwechselpigmente mit einer D50-Teilchengröße von mindestens 60 µm bis zu 150 µm enthält, um den Farbwechseleffekt nach Auftragen auf die Haut beizubehalten, und wobei die Farbwechselpigmente Substrate enthalten, die aus der Gruppe bestehend aus natürlichem und/oder synthetischem Glimmer, Aluminiumoxiden, Glasplättchen, SiO₂-Plättchen, Talkum, Kaolin, Sericit, plättchenförmigen Eisenoxiden oder TiO₂-Plättchen, Graphitplättchen und BiOCl, die mit alternierenden Lagen aus hochbrechenden Beschichtungen mit einem Brechungsindex von n > 1,8 und niedrigbrechenden Beschichtungen mit einem Brechungsindex von n ≤ 1,8 beschichtet sind, ausgewählt sind.

2. Kosmetische Zubereitung nach Anspruch 1, die 1-20 Gew.-% der großkörnigen Farbwechselpigmente enthält.

3. Kosmetische Zubereitung nach einem der Ansprüche 1 bis 2, wobei es sich bei den alternierenden Schichten um Metalloxide handelt.

4. Kosmetische Zubereitung nach Anspruch 3, wobei es sich bei den Metalloxiden um TiO₂ und SiO₂ oder Fe₂O₃ handelt.

5. Kosmetische Zubereitung nach einem der Ansprüche 1 bis 4, wobei die großkörnigen Farbwechselpigmente mehr als 2 Metalloxidschichten enthalten.

6. Kosmetische Zubereitung nach einem der Ansprüche 3 bis 5, wobei die Metalloxidschichten TiO₂-SiO₂-TiO2 sind.

7. Kosmetische Zubereitung nach einem der Ansprüche 4 bis 6, wobei es sich bei dem TiO₂ um Rutil handelt.

8. Kosmetische Zubereitung nach einem der Ansprüche 1 bis 7, wobei die Pigmente mit Farbwechseleffekt weiterhin als Deckschicht ein Absorptionspigment oder einen wasserunlöslichen Farbstoff/Farblack enthalten.

9. Kosmetische Zubereitung nach Anspruch 8, die als Deckschicht ein Eisen(II)-hexacyanoferrat(II), Berliner Blau, Karminrot, Indigo, oder Karmin, FD&C-Farbstoffe/Farblacke, D&C-Farbstoffe/Farblacke enthält.

10. Farbwechselpigment mit einer D50-Teilchengröße von mindestens 60 µm bis zu 150 µm und Substraten, die aus der Gruppe bestehend aus natürlichem und/oder synthetischem Glimmer, Aluminiumoxiden, Glasplättchen, SiO₂-Plättchen, Talkum, Kaolin, Sericit, plättchenförmigen Eisenoxiden oder TiO₂-Plättchen, Graphitplättchen und BiOCl, die mit alternierenden Lagen aus hochbrechenden Beschichtungen mit einem Brechungsindex von n > 1,8 und niedrigbrechenden Beschichtungen mit einem Brechungsindex von n ≤ 1,8 beschichtet sind, ausgewählt sind.

11. Farbwechselpigment nach Anspruch 10 mit einem D50-Bereich von 75-90 µm.

12. Farbwechselpigment nach einem der Ansprüche 10 oder 11, das als Deckschicht ein Absorptionspigment, einen wasserunlöslichen Farbstoff oder einen wasserunlöslichen Farblack enthält.

13. Kosmetische Zubereitung nach einem der Ansprüche 1-9 in Form eines Augenkonturenstiftes oder Eye Highlighters, einer Creme to Powder, einer Grundierung oder eines Bronzers in flüssiger oder Stift-Form, eines Lipglosses, eines Lippenstiftes, eines Nagellacks, eines Eyeliners, einer Wimperntusche, eines Haargels, eines Duschgels, einer Körperlotion, einer Hautcreme oder eines Shampoos.

## Revendications

1. Composition cosmétique contenant des pigments ayant un effet de voyage de la couleur, où ladite composition contient 0,01-35% en poids de pigments à voyage de la couleur et de taille de particules importante, ayant une taille de particules D50 allant d'au moins 60 µm jusqu'à 150 µm afin de conserver l'effet de voyage de la couleur lors d'une application à la peau, et où les pigments à voyage de la couleur comprennent des substrats choisis dans le groupe constitué par le mica naturel et/ou synthétique, les oxydes d'aluminium, les paillettes de verre, les paillettes de SiO₂, le talc, le kaolin, la séricite, les oxydes de fer sous forme de paillettes ou les paillettes de TiO₂, les paillettes de graphite, et le BiOCl, revêtus de couches alternées de revêtements à indice de réfraction élevé ayant un indice de réfraction n > 1,8, et de revêtements à indice de réfraction faible ayant un indice de réfraction n ≤ 1,8.

2. Composition cosmétique selon la revendication 1, contenant 1-20% en poids de pigments à voyage de la couleur et de taille de particules importante.

3. Composition de revêtement cosmétique selon l'une quelconque des revendications 1 à 2, dans laquelle les couches alternées sont des oxydes métalliques.

4. Composition cosmétique selon la revendication 3, dans laquelle les oxydes métalliques sont TiO₂ et SiO₂ ou Fe₂O₃.

5. Composition cosmétique selon l'une quelconque des revendications 1 à 4, dans laquelle les pigments à voyage de la couleur et de taille de particules importante contiennent plus de 2 couches d'oxydes métalliques.

6. Composition cosmétique selon l'une quelconque des revendications 3 à 5, dans laquelle lesdites couches d'oxydes métalliques sont TiO₂-SiO₂-TiO₂.

7. Composition cosmétique selon l'une quelconque des revendications 4 à 6, dans laquelle le TiO₂ est le rutile.

8. Composition cosmétique selon l'une quelconque des revendications 1 à 7, dans laquelle les pigments ayant un effet de voyage de la couleur comprennent en outre, comme couche supérieure, un pigment d'absorption ou un(e) colorant/laque insoluble dans l'eau.

9. Composition cosmétique selon la revendication 8, comprenant comme couche supérieure un ferrocyanure ferrique, du bleu de Prusse, du rouge carmin, de l'indigo ou du carmin, des colorants/laques FD&C, des colorants/laques D&C.

10. Pigment à voyage de couleur ayant une taille de particules D50 allant d'au moins 60 µm jusqu'à 150 µm, et des substrats choisis dans le groupe constitué par le mica naturel et/ou synthétique, les oxydes d'aluminium, les paillettes de verre, les paillettes de SiO₂, le talc, le kaolin, la séricite, les oxydes de fer sous forme de paillettes ou les paillettes de TiO₂, les paillettes de graphite, et le BiOCl, revêtus de couches alternées de revêtements à indice de réfraction élevé ayant un indice de réfraction n > 1,8, et de revêtements à indice de réfraction faible ayant un indice de réfraction n ≤ 1,8.

11. Pigment à voyage de couleur selon la revendication 10, ayant une plage de D50 de 75-95 µm.

12. Pigment à voyage de couleur selon l'une quelconque des revendications 10 ou 11, où ledit pigment comprend, comme couche supérieure, un pigment d'absorption, un colorant insoluble dans l'eau ou une laque insoluble dans l'eau.

13. Composition cosmétique selon l'une quelconque des revendications 1-9, sous la forme d'un crayon pour les yeux ou surligneur pour les yeux, une crème à poudre, un fond de teint ou agent bronzant liquide ou en bâtonnet, un brillant à lèvres, un rouge à lèvres, un vernis à ongles, un eyeliner, un mascara, du gel pour cheveux, un gel de douche, une lotion pour le corps, une crème pour la peau ou un shampooing.
